Europäisches Patentamt

European Patent Office

Office européen des brevets

(19)

(11) Veröffentlichungsnummer: **0 334 989**
**A2**

## (12) EUROPÄISCHE PATENTANMELDUNG

(21) Anmeldenummer: **88107113.8**

(22) Anmeldetag: **04.05.88**

(51) Int. Cl.⁴: **A61F 2/66**

(30) Priorität: **29.03.88 DE 8804228 U**

(43) Veröffentlichungstag der Anmeldung:
**04.10.89 Patentblatt 89/40**

(84) Benannte Vertragsstaaten:
**AT BE CH DE ES FR GB IT LI LU NL SE**

(71) Anmelder: **IPOS GMBH & CO. KG.**
**Lüner Rennbahn 14**
**D-2120 Lüneburg(DE)**

(72) Erfinder: **Prahl, Gregor M.**
**Nutzfelder Weg 21**
**D-2127 Rullstorf(DE)**

(74) Vertreter: **Richter, Werdermann & Gerbaulet**
**Neuer Wall 10**
**D-2000 Hamburg 36(DE)**

(54) **Kunstfuss für eine Beinprothese.**

(57) Der Kunstufuß für eine Beinprothese weist in seinem Sohlenbereich einen in den Fußformteil eingebetteten, plattenförmigen Versteifungskörper (10) auf, der aus zwei etwa gleich langen, übereinanderliegenden Blattfedern (10a,10b) besteht, die dem Abrollprofil des Fußes entsprechend geformt sind, wodurch eine verbesserte Mittelfußelastizität erreicht wird.

*FIG. 1*

EP 0 334 989 A2

## Kunstfuß für eine Beinprothese

Die Erfindung betrifft einen Kunstfuß für eine Beinprothese aus einem Schaumkunststoff-Fußformteil mit einem aus einem in seinem Sohlenbereich eingebetteten plattenförmigen, metallischen Versteifungskörper, der im Ballenbereich mit einer fußballengleich verlaufenden Kröpfung zur Unterstützung der Fußabrollfunktion ausgebildet ist und mit - jeweils als elastisch nachgiebige Abfederung dienend - einem Vorfußkern und einer im Fersenbereich angeordneten Lasche bestehenden Funktionskern, wobei der Funktionskern und der Versteifungskörper fest miteinander verbunden sind.

Als Material für Kunstfüße wird seit langem Polyurethan-Schaumkunststoff eingesetzt, der den Vorteil eines niedrigen Gewichtes aufweist.

Damit der Kunstfuß die bei einem natürlichen Fuß vorgegebene Funktion annähernd erfüllen kann, ist z.B. in der US-PS 3,335,428 ein Bein-Prothesen-Fußteil vorgeschlagen worden, der aus elastischen Kunststoffen unterschiedlicher Härte geformt ist.

Ferner ist aus der DE-PS 354 246 ein Kunstfuß bekannt, bei dem im Sohlenbereich des Kunstfußes eine Metallschiene eingebettet ist. Dieser künstliche Fuß soll das Weitausschreiten eines Fuß- oder Beinamputierten ermöglichen,da das Weitausschreiten das Schrägnachhintenverlegen des Unterschenkels, also das Nachgeben der Ferse des mit seiner ganzen Unterfläche auf dem Boden aufruhenden Fußes bedingt. Er soll ferner das leichte Wiederaufrichten des Unterschenkels bedingen, ohne daß dieser die Senkrechtlage überschreitet, während das Verlegen des Körpergewichtes nach vorn und das Anheben der Ferse durch die übliche Nachgiebigkeit des Mittelfuß-und Zehenteils des Kunstfußes ermöglicht werden soll. Diese Nachgiebigkeit soll durch Einlagern einer durch den Sohlen- und Fersenteil hindurchgehenden Federplatte erzielt werden. Um nunmehr die Ausladung des Unterschenkels schräg nach hinten herbeiführen zu können, sieht diese bekannte Ausführungsform eines Kunstfußes eine Verbindung zwischen dem starren Unterschenkel und der Federplatte vor,bei der sich der Schenkel in Art einer Wiege aus seiner Aufrechtstellung nach rückwärts bewegt und bei Verlegung des Körpergewichtes nach vorne wieder in die erste übergeht. Diese Schaukelbewegung erfolgt dadurch, daß der keilförmig zulaufende, starre Unterschenkel in einem Sattel sitzt, dessen eine Schräge vom Spann des Kunstfußes und dessen Gegenschräge von einer Abzweigung der Federplatte gebildet wird. Zur weiteren Abfederung befindet sich zwischen der Abzweigung und der eigentlichen Federplatte ein zusammendrückbares Keilkissen, z.B. aus weichem Gummi. Damit beim Anstoßen der vorderen Keilfläche am Schenkel gegen den Fuß kein unerwünschtes Geräusch auftritt, besteht der Vorder-bzw. Mittelfuß aus einer schmiegsamen oder leicht zusammendrückbaren, jedenfalls geräuschdämpfenden Masse, vorzugsweise Filz. Mit einer Kappe aus diesem Stoff ist auch die Widerlagerfläche des Unterschenkels überzogen. Die Abzweigung der Federplatte ist bei diesem künstlichen Fuß mit der hinteren Keilfläche des Unterschenkels fest verbunden. Dabei wird jedoch die Abzweigung nicht scharf abgebogen, da eine wiegende oder schaukelnde Bewegung angestrebt wird. Sie ist daher mit ihrem Knie gleitend auf der Platte angebracht.

Bei diesem bekannten Kunstfuß ist somit eine Stahlfeder mit federnden Eigenschaften eingesetzt, die bis in den Vorfuß reicht. Die wichtigen Elastizitätsunterschiede zwischen Mittelfuß und dem Vorfuß sind hier jedoch konstruktiv nicht berücksichtigt. Die verwendete Stahlfeder ermöglicht jedoch kein natürliches Abrollen, auch sind die Verbindungsprobleme zwischen den verschiedenen Materialien im elastischen Bereich nicht konstruktiv gelöst.

Es ist ferner aus der DE-PS 361 972 ein künstlicher Fuß mit einer aus mehreren,gegeneinander abgestuften Blattfedern bestehenden Längsfederung bekannt, deren Blattfedern mit ihren rückwärtigen Enden auf der unteren Seite eines den hinteren Fußteil bildenden, aber von der Sohle durch eine Zwischenschicht getrennten starren Klotzes befestigt sind, während die vorderen, in geeigneter doppelter Krümmung abwärts geführten Federn den unmittelbar auf die zweckmäßig durch ein Schutzblech bedeckte Sohle aufdrücken. Auch bei diesem künstlichen Fuß wird versucht, die Fußbewegungen über eine Metallfeder zu steuern; sie läßt jedoch keine Beweglichkeit im Zehengrundbereich zu.

Bei einem aus der US-PS 2,556,525 bekannten Kunstfuß für Beinprothesen ist in einem äußeren Schaumkunststoff-Fußformteil ein steifer, jedoch flexibler Kunststoffteil eingelassen, der sich über die gesamte Länge des Fußes erstreckt, wobei in diesem steifen, jedoch flexiblen Kunststoffteil eine Metalleinlage aus Federstahl eingebettet ist. Auch wenn ein innerer, teilweise flexibler Kunststoffteil mit einer Metalleinlage aus Federstahl bei diesem bekannten Kunstfuß verwendet wird, so ist es bei diesem Kunstfuß nicht möglich, die Abrollfunktion auf die von der Natur vorgegebene Drittelungslinie zu legen.

Im übrigen ist auch die Metalleinlage bis in die Fußspitze geführt. Trotz der Verwendung eines flexiblen Kunststoffteiles und einer Metalleinlage aus Federstahl ist eine ausreichende Beweglichkeit im Zehengrundgelenk, wie dies in der Natur gegeben ist, nicht möglich. Das wichtige Einknicken im Zehengrundgelenk ist bei dieser bekannten Ausführungsform nicht berücksichtigt und aufgrund der konstruktiven Ausgestaltung auch nicht möglich.

Um einen Kunstfuß für Beinprothesen mit einem über einen längeren Zeitraum vorgegebenen Abrollwiderstand hoher Elastizität und einer Einknickmöglichkeit im Zehengrundgelenk zu schaffen, ist daher in der DE-PS 23 41 887 der eingangs beschriebene Kunstfuß vorgeschlagen worden. Bei diesem Kunstfuß wird die Vorfußelastizität durch Einsatz eines homogenen Vulkollanteils (Elastomer) gewährleistet, d.h., es wird im Vorfuß eine Rückstellelastizität berücksichtigt, die weit über bis dahin geformte Polstereffekte hinausging. Allerdings war man bisher nicht in der Lage, die Vorteile dieser Rückstellelastizität in ihrer physikalischen Einflußgröße auf das Gangbild funktionell zu nutzen.

Es ist daher Aufgabe der vorliegenden Erfindung, den eingangs genannten Fuß dergestalt weiterzuentwickeln, daß eine große Mittelfußelastizität bis zu einem maximalen Abrollmoment von 120 Nm auch bei Dauerbeanspruchung von bis zu 3 Millionen Lastwechseln gewährleistet ist, ohne auf die Vorteile des aus der DE-PS 23 41 887 bekannten Kunstfußes zu verzichten.

Diese Aufgabe wird dadurch gelöst, daß der Versteifungskörper aus mindestens zwei etwa gleich langen, übereinanderliegenden Blattfedern besteht, die dem Abrollprofil des Fußes entsprechend geformt sind.

Vorteilhafterweise weist dieser Fuß eine große Beweglichkeit im Zehengrundgelenk auf, ferner eine elastische, nachgiebige Abfederung sowohl im Vorderfuß als auch im Fersenbereich, wobei der Abrollwiderstand für einen längeren Zeitraum unveränderlich vorgegeben ist. Darüber hinaus ist eine einfache Anpassung an unterschiedliche Absatzhöhen möglich und für den Träger des Kunstfußes ein Höchstmaß an Tragekomfort gewährleistet, ohne daß Nachstellfunktionen zur Beibehaltung einer gleichmäßigen Fußabrollfunktion erforderlich werden. Durch die Abstützung des metallischen Versteifungskörpers im Fersenteil des Fußformteils wird eine erhöhte Festigkeit des Fußformteils erreicht, ohne daß die erforderlichen elastischen und nachgiebigen Abfederungen im Vorderfuß und im Fersenbereich beeinträchtigt werden. Die einfache Anpassung an unterschiedliche Absatzhöhen ist lediglich durch Veränderung der im Fersenteil des Fußformteils angeordneten Lasche mühelos möglich, ohne daß hierfür eine Veränderung des gesamten Funktionskernes erforderlich ist, um eine Anpassung an unterschiedliche Absatzhöhen zu erreichen.

Insbesondere ermöglicht die erfindungsgemäße Konstruktion, daß durch die federnd gestalteten Vor- und Mittelfußbereiche Bewegungsenergie aufgenommen, gespeichert und in der Entlastung der Schrittrücklage wieder als frei werdende Energie in die Prothese eingespeist wird. Diese Rückführung frei gewordener Bewegungsenergie in die prothetischen Systeme führt nachweislich zu einer deutlichen Energieentlastung während der Fortbewegung des Patienten. Ferner ist es nunmehr möglich, auf Fußgelenke in dem Kunstfuß zu verzichten, wobei ein reibungsbedingt in den Gelenken entstehender Energieverlust vermieden und die gespeicherte Energie voll als Rückführung wieder nutzbar gemacht werden kann Der Kunstfuß ist im Vor- und Mittelfußbereich hochfederelastisch und kann ein maximales Abrollmoment von 120 Nm aufnehmen Diese maximale Abrollbelastung bestimmt auch die Federdimensionierung.

Weiterbildungen der Erfindung sind in den Unteransprüchen 2 bis 11 beschrieben.

Insbesondere ist hierbei die hochfederelastische Wirkung der als Doppelblattfedern wirkenden Blattfedern aus Titan, einer hochfesten Titan-Aluminiumlegierung oder einem geeigneten, vorzugsweise gleiche Eigenschaften wie Titan aufweisenden Werkstoff hervorzuheben, die nach einer Weiterbildung der Erfindung durch den dazwischen angeordneten Abstandshalter eine Kontaktberührung der jeweiligen Blattfederoberflächen verhindert. Der Abstandshalter besteht vorzugsweise aus hochmolekularem Polyäthylen oder einem anderen geeigneten Kunststoff und wirkt als geräuschdämpfende Gleitschicht zur Steuerung der maximal aufzunehmenden Federkraft.

Ein Ausführungsbeispiel der Erfindung soll anhand der Zeichnungen im Nachfolgenden erläutert werden. Es zeigen

Fig. 1 einen Schnitt durch den Kunstfuß,

Fig. 2 eine Seitenansicht des aus zwei Blattfedern bestehenden Versteifungskörpers und

Fig. 3 eine Draufsicht auf die untere Blattfeder mit aufgelegtem Abstandshalter.

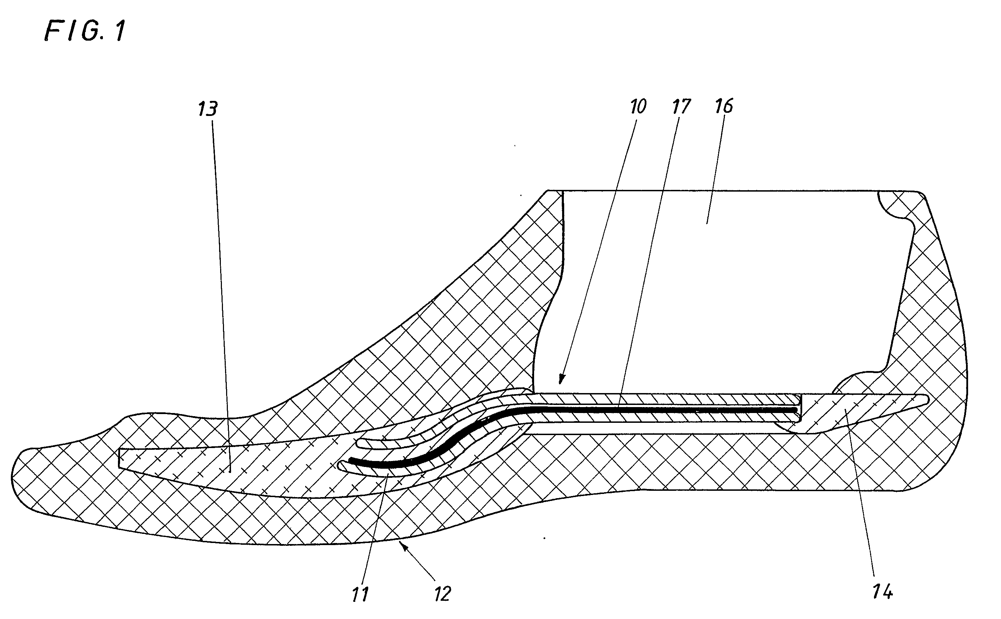

Der in Fig. 1 dargestellte Versteifungskörper 10 besitzt eine nach unten gerichtete Kröpfung 11 im Ballenbereich 12 des Kunstfußes, die dem Abrollprofil des Fußes entsprechend geformt ist. Der Versteifungskörper 10 ist in einem Vorfußkern 13 ebenso eingebettet wie in einer im Fersenbereich liegenden Lasche 14. Der aus den genannten Teilen 10, 13 und 14 bestehende Funktionskern ist von einem Schaumkunststoff-Fußformteil 15 umschäumt, das im rückwärtigen Teil einen Hohlraum 16 aufweist, der bis auf den Versteifungskörper 10 geführt ist und in dem Gelenke oder starre Befestigungselemente direkt an dem Versteifungskörper 10 verschraubt werden können.

Die nach unten gerichtete Kröpfung 11 ist dergestalt ausgebildet, daß die Abrollfunktion auf die von der Natur vorgegebene vordere Drittelungslinie (Zehengrundgelenklinie) gelegt ist. Dabei ist an der Kröpfung 11 des Versteifungskörpers 10 ein aus einem Elastomer bestehender Vorfußkern 13 anvulkanisiert, der in seiner unteren Abschlußlinie der äußeren Form des Fußballens entspricht. Dieser Vorfußkern 13 ist durch eine umgeschäumte Struktur völlig alterungsbeständig, so daß der Abrollwiderstand über längere Zeiträume unveränderlich vorbestimmbar ist. Gleichfalls ist im Fersenteil zur Verstärkung des Versteifungskörpers 10 ebenfalls aus einem Elastomer eine Lasche 14 anvulkanisiert.

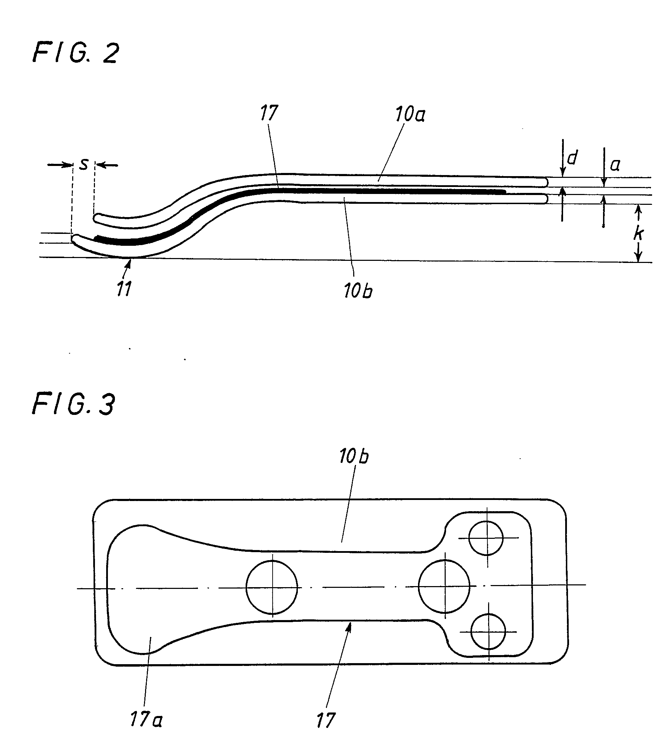

Der die Erfindung insbesondere betreffende Aufbau des Versteifungskörpers 10 ist aus den Fig. 2 und 3 im Detail ersichtlich. Die Mittelfußkonstruktion besteht hierbei aus zwei etwa gleich langen Blattfedern 10a und 10b, die in übereinanderliegender Anordnung die bereits beschriebene Formgestaltung des Versteifungskörpers 10 besitzen. Auch mehr als zwei Blattfedern können zur Anwendung gelangen. Im rechtsseitigen Bereich sind die Blattfedern 10a und 10b parallellaufend eben ausgebildet, wobei ein dazwischen liegender Abstandshalter aus Polyäthylen oder einem anderen geeigneten Werkstoff einen Kontakt der Oberflächen der Blattfedern 10a,10b verhindert.

Der Abstandhalter ist im rechtsseitigen Bereich (Fig.3) im wesentlichen T-förmig ausgebildet, wobei dessen zum Vorderfuß ausgerichteter Schenkel eine zungenförmige Verbreiterung erfährt, vorzugsweise dergestalt, daß die Blattfedern 10a und 10b den Abstandhalter 17 allseitig überragen.

Sind mehrere Blattfedern zum Versteifungskörper 10 zusammengefaßt, dann ist jeweils zwischen je zwei Blattfedern ein Abstandshalter 17 angeordnet.

Der Abstandhalter dient insbesondere als geräuschdämpfende Gleitschicht, durch deren Stärke die maximal aufzunehmende Federkraft gesteuert werden kann. Je größer der Abstand a der Blattfedern 10a und 10b ist, desto größer ist die maximale Belastbarkeit des Kunstfußes. Zum Vorderteil hin, also in den Fig. 2 und 3 linksseitig, wird durch die der Abrollform entsprechende Kröpfung ein breiter werdender Blattfederspalt erreicht. Die untere Feder 10b überragt vorderseitig die obere Blattfeder 10a um eine Abstand s von ca. 5 mm. Die in Fig. 2 und 3 dargestellte Blattfederanordnung mit zwischengelegtem Abstandhalter wird in einer Form entsprechend der Fußgröße und Fußseite in ein homogenes Funktionsteil eingegossen. Hierbei wird berücksichtigt, daß die Vorfußelastizität und die Mittelfußelastizität fließend ineinander übergehen.

Um die hohe Gleiteigenschaft des Abstandhalters 17 zu erreichen, kann dieser auch aus Polyurethane, insbesondere einem vernetzten Polyurethan-Elastomer, das unter dem Handelsnamen Vulkollan bekannt ist, bestehen. Polyurethan weist eine hohe Abriebfestigkeit auf, so daß immer ein gleichbleibender Abrolleffekt gewährleistet ist. Hinzu kommt das hohe , selbständige Rückstellvermögen aus einer verformten Stellung in die Ausgangsstellung. Auch der Fußkern 13 und die Lasche 14 bestehen aus Polyurethan, insbesondere einem vernetzten Polyurethan-Elastomer, das unter dem Handelsnamen Vulkollan bekannt ist. Auch Polyurethane, die durch Umsetzung von Diisocyanaten, die unter dem Handelsnamen Desmodur bekannt sind, nach dem Polyisocyanat-Polyadditonsverfahren erhalten werden, können eingesetzt werden. Dabei häufig eingesetzte Isocyanat-Typen sind TDI,MDI und HDI. Für stark vernetzte Polyurethane kommen vor allem Tri- und u.a. auch Polyisocyanate zum Einsatz. Auch hier wird die Eigenschaft einer hohen Gleitfähigkeit ausgenutzt.

Durch die im Bereich der Kröpfung vorhandene Biegerate-Differenz der oberen und der unteren Blattfeder 10a, 10b werden die Horizontalverschiebungen der beiden Federteile durch den breiter werdenden Elastomeranteil (Polyurethan) des Abstandshalters 17 kompensiert. Der umlaufend um den Abstandshalter 17 vorhandene Restspalt läuft mit dem Polyurethan auf, d.h., zwischen den beiden Blattfedern 10a und 10b, die durch besondere Haftvermittler mit dem Polyurethan verwachsen, entsteht eine elastische Verbindung, die durch den Abstand k zwischen der durch die hintere Blattfederebene und der Kröpfung 11 Federwege am vorderen Belastungteil von mehr als 15 mm im Abrollpunkt ermöglicht. Diese relativ großen Federwege machen zum Teil den Einsatz von Gelenken durch ihre funktionell große Dorsalflexion überflüssig. Gleichzeitig entsteht bei der Rückfederung ein großer Bereich frei werdender Energie, die in das Prothesensystem wieder eingespeist werden kann.

Die Blattfederkonstruktion ist bei minimalem Gewicht einer hohen Abrollbelastung auf Dauer gewachsen, arbeitet völlig geräuschfrei und zeichnet sich durch große Federwege aus. Aufgrund der für die Blattfeder gewählten hochfesten Titanlegierung in Verbindung mit Polyurethan, in welchem die Blattfederanordnung eingebettet ist, ist der erfindungsgemäße Kunstfuß anderen Blattfederkonstruktionen, wie sie im Bereich von kohlefaserverstärkten Systemen bekannt sind, weit überlegen. Insbesondere waren bei bisher nach dem Stand der Technik bekannten Konstruktionen derart große Federwege nicht möglich.

## Ansprüche

1. Kunstfuß für eine Beinprothese aus einem Schaumkunststoff-Fußformteil mit einem aus einem in seinem Sohlenbereich eingebetteten plattenförmigen,metallischen Versteifungskörper, der im Ballenbereich mit einer fußballengleich verlaufenden Kröpfung zur Unterstützung der Fußabrollfunktion ausgebildet ist, und mit - jeweils als elastisch nachgiebige Abfederung dienend - einem Vorfußkern und einer im Fersenbereich angeordneten Lasche bestehenden Funktionskern, wobei der Funktionskern und der Versteifungskörper fest miteinander verbunden sind, dadurch gekennzeichnet, daß der Versteifungskörper (10) aus mindestens zwei etwa gleich langen, übereinanderliegenden Blattfedern (10a,10b) besteht, die dem Abrollprofil des Fußes entsprechend geformt sind.

2. Kunstfuß nach Anspruch 1, dadurch gekennzeichnet, daß die Blattfedern (10a,10b) aus Titan, einer hochfesten Ti-Al-Legierung od.dgl. bestehen.

3. Kunstfuß nach Anspruch 1 oder 2, dadurch gekennzeichnet, daß die Blattfedern gleich dick sind, vorzugsweise eine Dicke (d) von 2,7 mm aufweisen.

4. Kunstfuß nach einem der Ansprüche 1 bis 3, dadurch gekennzeichnet, daß die untere Blattfeder (10b) die obere (10a) zur Fußspitze hin um mindestens 5 mm (Strecke s) überragt.

5. Kunstfuß nach einem der Ansprüche 1 bis 4, dadurch gekennzeichnet, daß der Versteifungskörper (10) bzw. die Blattfedern (10a,10b) in einer Draufsichtprojektion im wesentlichen rechteckig sind, d.h. abgerundete Ecken und Kanten besitzen.

6. Kunstfuß nach einem der Ansprüche 1 bis 5, dadurch gekennzeichnet, daß zwischen den Blattfedern (10a,10b) ein elastischer Abstandshalter (17), vorzugsweise aus eine hohe Gleitfähigkeit aufweisendem, insbesondere hochmolekularem Polyäthylen oder einem anderen geeigneten Werkstoff angeordnet ist.

7. Kunstfuß nach einem der Ansprüche 1 bis 6, dadurch gekennzeichnet, daß der Abstand (a) der Blattfedern (10a,10b) im Fersenbereich mindestens 1 mm beträgt und/oder zur Fußspitze hin größer wird, vorzugsweise mehr als 3,5 mm, weiterhin vorzugsweise zwischen 3,5 mm und 4 mm liegt.

8. Kunstfuß nach Anspruch 6 oder 7, dadurch gekennzeichet, daß der Abstandshalter (17) im Fersenbereich 1 mm und zur Fußspitze hin 3,5 bis 4,5 mm dick ist.

9. Kunstsfuß nach einem der Ansprüche 6 bis 8, dadurch gekennzeichnet, daß der Abstandshalter im Fersenbereich im wesentlichen T-förmig ausgebildet ist und der zur Fußspitze hin gerichtete Schenkel (17a) zungenförmig breiter wird, vorzugsweise dergestalt, daß die Blattfedern (10a,10b) den Abstandshalter (17) an allen Seiten überragen.

10. Kunstfuß nach einem der Ansprüche 1 bis 9, dadurch gekennzeichnet, daß die Blattfedern in den Fußkern (13) und die Lasche (14) eingebettet sind, die beide aus Polyurethan, insbesondere einem vernetzten Polyurethan-Elastomer bestehen.

11. Kunstfuß nach einem der Ansprüche 1 bis 10, dadurch gekennzeichnet, daß die Blattfedern (10a,10b) vom Fersenbereich bis in den Mittelfußbereich im wesentlichen eben ausgebildet sind und die Kröpfung (11) im Ballenbereich (12) mindestens 15 mm, vorzugsweise 16 bis 21 mm tiefer verläuft (Abstand k).

FIG.1

*FIG. 2*

*FIG.3*